# EUROPÄISCHE PATENTSCHRIFT

(11) **EP 1 285 266 B1**
(45) Veröffentlichungstag und Bekanntmachung des Hinweises auf die Patenterteilung: **08.09.2004**
(21) Anmeldenummer: 01943405.9
(22) Anmeldetag: 18.05.2001
(51) Int. Cl.: G01N 33/00

(54) **Verfahren zur Messung des SO2-Gehalts in Wein**
Process for determining SO2 content in wine
Procédé de mesure du contenu de SO2 dans le vin

(30) Priorität: 22.05.2000 DE 10024947
(43) Veröffentlichungstag der Anmeldung: 26.02.2003
(73) Patentinhaber: Crinotec E.K., 72076 Tübingen (DE)
(72) Erfinder: FIEDLER, Andreas, 72070 Tübingen (DE); PFEFFERLE, Christoph, 72581 Dettingen an der Erms (DE)
(74) Vertreter: Otten, Hajo, Dr.-Ing.
(86) Internationale Anmeldenummer: PCT/EP2001/005715
(87) Internationale Veröffentlichungsnummer: WO 2001/090742

(56) Entgegenhaltungen:
- FR-A- 2 599 846
- US-A- 4 404 287
- PATENT ABSTRACTS OF JAPAN vol. 017, no. 314 (P-1557), 15. Juni 1993 (1993-06-15) & JP 05 034331 A (MEIDENSHA CORP), 9. Februar 1993 (1993-02-09)

## Beschreibung

Die Erfindung betrifft ein Verfahren zur Bestimmung des frei und/oder an Inhaltsstoffe gebunden SO₂ in Wein gemäß Anspruch 1, bei dem aus einem Gasraum über der Probe die Komponente in der Gasphase gemessen wird. Bevorzügte Ausführungsbeispiel des Verfahrens sind in den abhängigen Ansprüche definiert.

Ein Verfahren der eingangs genannten Art, das zur Messung des SO₂-Gehaltes in Wein oder Fruchtsäften dient, ist beschrieben in C. S. Ough: "Determination of Sulfur Dioxide in Grapes and Wines", veröffentlicht in Linskens & Jackson: Modern Methods of Plant Analysis, Band 6: Weinanalysis, Springer Verlag 1988.

Schwefeldioxid (SO₂) ist sicherlich die am häufigsten in Wein analysierte und gemessene Komponente. Aber auch in Fruchtsäften oder in anderen Nahrungsmitteln auch fester Konsistenz ist SO₂ eine aus gesundheitlichen Gründen nicht zu vernachlässigende Komponente.

Darüber hinaus findet SO₂ in der Umweltanalytik Beachtung, z.B. bei Kontrollsystemen der Abluft, Rauchgasentschwefelung, MAK-Bestimmung, etc.

Für viele Einsatzbereiche von SO₂ hat der Gesetzgeber inzwischen Grenzwerte erlassen, die von der Industrie, den Erzeugerund Abfüllbetrieben eingehalten und folglich regelmäßig kontrolliert werden müssen.

Ein Verzicht auf SO₂ z.B. bei der Weinherstellung ist häufig nur bis zu einem gewissen Maß möglich, denn SO₂ wird z.B. beim "Ausschwefeln" der Weinfässer eingesetzt, dient aber auch als Konservierungsstoff und wird allgemein als unverzichtbare Komponente für die qualitativ hochwertige Weinherstellung angesehen. Dennoch hat SO₂ gesundheitlich nachteilige Nebenwirkungen, die sich z.B. durch starke Kopfschmerzen nach dem Konsum von stark SO₂-haltigen Weinen manifestieren.

Darüber hinaus gibt es insbesondere bei Wein eine ganze Reihe von Weinfehlern und Weinkrankheiten, die durch unerwünschte oder zu hohe Konzentrationen von Komponenten verursacht werden. Hierzu zählt z.B. der durch Essigsäure hervorgerufene Essigstich, der durch Milchsäure hervorgerufene Milchsäurestich, der u.a. durch Schwefelwasserstoff hervorgerufene Böckser, der u.a. durch Methyl-Tetrahydronaphthalin hervorgerufene Kork-Muffton oder der durch 3-Methyl-gamma-octanolid hervorgerufene Holzton. Wegen weiterer Informationen zu Weinfehler und Weinkrankheiten verursachenden Komponenten siehe Römpp Lexikon Chemie, 10. Auflage, 1999, Georg Thieme Verlag, Stuttgart.

Im Wein kommt Schwefeldioxid bzw. die schweflige Säure sowohl frei als auch an verschiedene Inhaltsstoffe gebunden vor. Alle Zustandsformen zusammen geben die gesamte schweflige Säure oder SO₂-Konzentration, die auf bestimmte Maximalwerte hin überwacht werden muß. Die Entstehung von gebundenem SO₂ beruht auf der sogenannten Bisulfit-Addition an Aldehyde und Ketone.

Aus den gesetzlichen Untersuchungsvorschriften sowie entsprechenden Handbüchern für Winzer sind eine ganze Reihe von Meßverfahren bekannt, um die freie schweflige Säure oder das freie SO₂ zu bestimmen. Die bekannten Methoden sind einerseits sehr teuer und erfordern andererseits großen Arbeits- und Zeitaufwand. Durch viele Störparameter werden die Analysen ungenau und sind nicht gut reproduzierbar. Ein Problem ist dabei die in Wein sehr häufig vorhandene Ascorbinsäure, die z.B. bei dem Verfahren der Iodometrie, einem Titrierverfahren, fälschlich auch wie schweflige Säure erfaßt wird. Zur Ermittlung des wahren Wertes der freien schwefligen Säure muß die Ascorbinsäure gesondert bestimmt und vom erhaltenen Wert abgezogen werden.

Neben der Iodometrie ist es auch bekannt, SO₂ durch Colorimetrie, Oxidationsverfahren, Gasmeßelektroden, Polaritätsmessung, Reaktionen mit Pararosanilin, enzymatische Verfahren oder mittels Gaschromatographie zu bestimmen.

Damit bei den bekannten Verfahren auch die gebundene schweflige Säure mit bestimmt werden kann, wird diese durch Verseifung mit Lauge oder in der Hitze durch Destillation mit starken Säuren freigesetzt, was weitere, kostspielige und zeitaufwendige Verfahrensschritte beinhaltet, die zudem quantitativ nicht hinreichend zuverlässig sind.

Das von C. S. Ough a.a.O. beschriebene bekannte Verfahren findet sich dort in Kapitel 4 unter "Gaschromatographie" mit dem Stichwort "Headspace Analysis". Mit Hilfe eines Flammenphotometers oder eines elektrolytischen Hall Detektors wird die SO₂-Konzentration im Gasraum über der flüssigen Probe gemessen, wobei unter der Anwendung des Henry'schen Gesetzes dann die Konzentration der schwefligen Säure in Lösung berechnet wird. Das Henry'sche Gesetz, auch Henry'sches Absorptionsgesetz genannt, bringt zum Ausdruck, daß der Dampfdruck eines gelösten Stoffes proportional zu seinem Molenbruch in einer ideal verdünnten Lösung ist. Die Proportionalität wird durch eine empirische, temperaturabhängige Henrykonstante angegeben.

Obwohl die Messung mit anschließender Rechnung zum zufriedenstellenden Ergebnis führt, wird die Verwendung eines Hall Detektors oder eines Flammenphotometers jedoch als begrenzend für das angegebene Verfahren angegeben. Sowohl die Kosten als auch die erforderliche experimentelle Erfahrung für den Einsatz der angegebenen Detektoren begrenzen ihre Anwendung auf große Fachbetriebe. Für kleinere Winzer, Abfüller oder Analyselaboratorien ist das bekannte Verfahren nicht rentabel einzusetzen.

Die Messung von Schwefeldioxid in der Gasphase für vor allem Umweltmessungen erfolgt z.B. unter Anwendung von Gassensoren, bei denen das Prüfgas über eine gasdurchlässige Membran zum Sensor gelangt. Diese Sensoren weisen laut Herstellerangaben jedoch keine spezifische Empfindlichkeit gegenüber z.B. Schwefeldioxid auf, sie sprechen vielmehr auch auf andere Prüfgase an, weisen also eine hohe Querempfindlichkeit auf. Sie besitzen ferner eine relativ lange Ansprechzeit. Aufgrund geringer Empfindlichkeit wurde diese Methode für analytische Zwecke, insbesondere im Bereich der Lebensmittel- und Genußmitteluntersuchungen bisher nicht angewendet.

FR-A-2599846 offenbart ein Verfahren zur Bestimmung des Schwefelgehalts in flüssigen Kohlenwasserstoffen basierend auf der Umwandlung des Schwefels zu SO₂ und Messungen der Fluoreszenz des SO₂ in der Gasphase.

Die insoweit beschriebenen Verfahren der Schwefeldioxidanalyse erfordern also einen großen zeitlichen und apparativen Aufwand, wobei besonders von Nachteil ist, daß andere Komponenten mitbestimmt werden, so daß diese gesondert gemessen und vom erhaltenen Meßwert abgezogen werden müssen.

Hodgson et al., "Electrochemical Sensor for the Detection of SO₂ in the Low-ppb Range", Anal. Chem. 1999, Band 71, Seiten 2831-2837, beschreiben einen elektrochemischen Sensor auf der Basis einer elektrochemischen Zelle. Der beschriebene Sensor ermöglicht die Detektion von sehr geringen Konzentrationen von atmosphärischem Schwefeldioxid.

Schiavon und Zotti, "Electrochemical Detection of Trace Hydrogen Sulfide in Gaseous Samples by Porous Silver Electrodes Supported on Ion-exchange Membranes (Solid Polymer Electrolytes)", Anal. Chem. 1995, Band 67, Seiten 318-232, beschreiben die Messung von H₂S Spuren in gasförmigen Proben mittels eines elektrochemischen Sensors. Die Probe wird dazu mittels einer Spritze in einen N₂-Gasstrom injiziert, der den Sensor durchspült.

Vor diesem Hintergrund liegt der vorliegenden Erfindung die Aufgabe zugrunde, das Verfahren der eingangs genannten Art derart weiterzubilden, daß es reproduzierbar und preiswert durchgeführt werden kann.

Bei dem bekannten Verfahren wird diese Aufgabe dadurch gelöst, daß die Konzentration der in der Gasphase befindlichen Komponente mittels eines Sensors, vorzugsweise einer elektrochemischen Zelle erfaßt und in ein elektrisches Signal umgewandelt wird.

Eine Vorrichtung, die zur Durchführung des Verfahrens gemäß Anspruch 1 geeignet ist, umfaßt einen Aufnahmeraum für die flüssige Probe, einen vorzugsweise selektiv auf die Komponente ansprechenden Sensor, vorzugsweise auf Basis einer elektrochemischen Zelle, und ein Gasleitungssystem, über das ein Trägergas aus dem Aufnahmeraum durch den Sensor leitbar ist.

Ein Sensor, insbesondere auf der Basis einer elektrochemischen Zelle, Kann eingesetzt werden, um die in der Gasphase befindliche Komponente im Gasraum über der flüssigen Probe zu messen und daraus die Gesamtkonzentration der Komponente in der flüssigen Probe zu bestimmen. Hier wird direkt der SO₂-Gehalt bestimmt, nicht ein Reaktionsprodukt von SO₂, so daß die Messung schnell, einfach und genau durchgeführt werden kann.

Durch eine Schüttel- oder Rührbewegung der flüssigen Probe kann dabei die Komponente in die Gasphase, und damit in den Gasraum oberhalb der Probe überführt und mit dem Sensor gemessen werden.

Derartige Sensoren sind in der Literatur vielfach beschrieben, siehe z.B. Hodgson et al., a.a.O. und Schiavon und Zotti, a.a.O. Diese Sensoren sind preiswerte Bauteile, die durch entsprechende Auslegung für die Messung unterschiedlichster Komponenten verwendet werden können.

Die in dem dabei vorzugsweise in dem Sensor eingesetzte elektrochemische Zelle umfaßt eine Membran, auf die eine für die Komponente sensitive Beschichtung aufgebracht wurde. Das die zu messende Komponente enthaltende Gas kommt in direkten Kontakt mit der Sensoroberfläche, wodurch eine sehr kurze Ansprechzeit des Sensors ermöglicht wird. Durch ein exakt einstellbares Redoxpotential kann man selektiv z.B. Schwefeldioxid in kleinsten Mengen nachweisen.

Wegen weiterer Informationen über elektrochemische Sensoren wird auf Hodgson et al. a.a.O. sowie auf die dort umfangreich zitierten Veröffentlichungen verwiesen.

Vorzugsweise wird das Trägergas über das Gasleitungssystem durch die flüssige Probe und dann durch den Sensor geleitet, so daß die flüssige Probe mit einem Trägergas begast wird.

Der Vorteil dieser Maßnahme liegt darin, daß durch die Begasung die Komponente sozusagen aus der Probe verdrängt wird, so daß die frei in der Probe vorhandene Komponente, z.B. das SO₂, quantitativ in dem Trägergas in der Gasphase vorliegt und das Meßsignal des Sensors in die Konzentration der freien Komponente in der flüssigen Probe umgerechnet werden kann.

Dabei ist es bevorzugt, wenn das Gasleitungssystem mit einer Gasquelle für das Trägergas verbunden ist.

Hier ist von Vorteil, daß ein inertes Trägergas oder ein aufgereinigtes Gas verwendet wird, um aus der flüssigen Probe die freie Komponente in die Gasphase zu überführen und der Messung zuzuführen.

Dabei ist es besonders bevorzugt, wenn das Gasleitungssystem eine Ventilschaltung umfaßt, über die die Gasquelle mit dem Sensor und der Sensor an seinem Ausgang mit einer Abluftleitung verbindbar ist, so daß die elektrochemische Zelle vor der eigentlichen Messung mit dem Trägergas gespült werden kann.

Hier ist von Vorteil, daß vor der Messung der Sensor mit einem von zu messenden Komponenten freien Trägergas gespült wird, so daß sämtliche Verunreinigungen von dem Sensor entfernt werden, die aufgrund einer vorhergehenden Messung dort ggf. noch vorhanden sind.

Weiter ist es bevorzugt, wenn das Gasleitungssystem eine Ventilschaltung umfaßt, über die ein Strom von Trägergas im Kreislauf durch die flüssige Probe leitbar ist, so daß das Trägergas sozusagen im Kurzschluß durch die flüssige Probe geleitet wird.

Hier ist von Vorteil, daß sich durch diese zirkulare Begasung der Probe vor der eigentlichen Messung ein Gleichgewicht zwischen Gasphase und Probe einstellen kann, so daß der in der folgenden Messung bestimmte Meßwert in die Konzentration von SO₂ in der Probe umgerechnet werden kann.

Weiter ist es bevorzugt, wenn die Ventilschaltung den Strom von Trägergas auch durch den Sensor leitet, so daß das Trägergas im Kurzschluß durch die flüssige Probe und die elektrochemische Zelle geleitet wird.

Hier ist von Vorteil, daß während der eigentlichen Messung die Gefahr der Konzentrationsänderung in der Probe verringert wird, denn der Gasstrom, der den Sensor verläßt, wird in die Probe rückgeführt.

Es ist hier ferner bevorzugt, wenn die Ventilschaltung einen vorzugsweise kleinen Teil des Trägergases durch den Sensor leitet und den Rest durch eine Bypassleitung zurück in den Aufnahmeraum leitet.

Wenn nur ein geringer Teil des Trägergases durch den Sensor geleitet wird, ist es nicht unbedingt erforderlich, auch diesen kleinen Anteil des Trägergases zurück in die Probe zu führen, um einer Konzentrationsänderung entgegenzuwirken.

Dabei ist es bevorzugt, wenn in dem Aufnahmeraum eine Begasungseinrichtung vorgesehen ist, deren Einlaß über die Ventilschaltung wahlweise mit einem Ausgang des Sensors oder einer Gasentnahmeöffnung des Aufnahmeraums verbindbar ist.

Die Begasungseinrichtung kann eine in die Probe eingeführte Fritte oder eine am Boden des Aufnahmeraumes vorgesehene Fritte umfassen, so daß vorteilhafterweise eine großflächige Begasung ermöglicht wird, was zu einem konstanten Gleichgewicht zwischen SO₂ in der Gasphase und freiem SO₂ in der Probe führt.

Allgemein ist es bevorzugt, wenn die Gasquelle über eine Vorrichtung zum Anfeuchten des Trägergases mit der Ventilschaltung verbunden ist, so daß das Trägergas vor dem Spülen der elektrochemischen Zelle befeuchtet wird.

Der angefeuchtete Standby-Gasstrom verhindert in vorteilhafter Weise ein Austrocknen des Sensors und stellt damit eine zuverlässige und reproduzierbare Messung sicher.

Es ist auch möglich, einen Sensor zu verwenden, bei dem das Gas durch eine Membran hindurch diffundiert und erst dann mit einer aktiven Oberfläche des Sensors in Berührung kommt, die direkt mit einem Elektrolyten in Kontakt steht. Eine solche Membran kann aus einem gasdurchlässigen Teflonmaterial bestehen, wobei der Elektrolyt 0,5 mol/l Schwefelsäure enthält. Es sind aber auch andere Elektrolyten, wie beispielsweise Salpetersäure, Perchlorsäure etc. verwendbar. Durch den direkten Kontakt der aktiven Oberfläche mit dem Elektrolyten wird ein Austrocknen der Oberfläche vermieden, so daß ein Befeuchten des Trägergases nicht erforderlich ist.

Allgemein ist es bevorzugt, wenn die Gasquelle eine Pumpe umfaßt, die Umgebungsluft ansaugt und als Trägergas in das Gasleitungssystem fördert, wobei vorzugsweise die Pumpe an ihrer Ansaugseite mit einem Gasfilter versehen ist, um die Bestimmung der Komponente störende Anteile aus dem Trägergas herauszufiltern. Auf diese Weise können aus dem Trägergas eventuell vorhandene Anteile, vorzugsweise der Komponente selbst ausgefiltert werden.

Bei dieser Maßnahme ist von Vorteil, daß eine günstige Gasversorgung geschaffen wird, die auch für kleine Labors ohne Gasflaschen realisierbar ist. Durch das Filter wird dabei sichergestellt, daß das Trägergas frei von z.B. SO₂ ist, so daß eine fehlerhafte Detektion, insbesondere im Hinblick auf eine mögliche Querempfindlichkeit des Sensors vermieden wird.

Weiter ist es bevorzugt, wenn der Aufnahmeraum mit einem Probenleitungssystem verbunden ist, über das die Probe in den Aufnahmeraum einfüllbar und mit einem Verdünnungsmedium vermischbar ist, so daß die Probe vor der Messung verdünnt wird.

Auf diese vorteilhafte Weise wird eine Übersättigung der Gasphase vermieden, wobei ferner auch das Volumen des Gasraumes oberhalb der Probe mit beeinflußt werden kann. Somit kann z.B. die Schwefeldioxidkonzentration an den Meßbereich des Sensors angepaßt werden, wobei auch das Volumen des Gasraumes diese Konzentration mit beeinflußt.

Weiter ist es bevorzugt, wenn das Probenleitungssystem eine Heiz-/Kühleinrichtung enthält, um die Probe und/oder das Verdünnungsmedium vor dem Einfüllen zu temperieren, wobei weiter vorzugsweise der Aufnahmeraum mit einem Heizsystem verbunden ist, vorzugsweise eine Heizwendel aufweist, um die flüssige Probe vor der Messung zu erhitzen.

Über das Probenleitungssystem kann auch der pH-Wert der flüssigen Probe vor der Messung in den sauren Bereich abgesenkt werden.

Durch die Ansäurung und/oder Erhitzung der Probe wird vorteilhaft zur Freisetzung z.B. des Schwefeldioxids beigetragen. An Inhaltsstoffe der Probe gebundenes Schwefeldioxid kann durch pH-Verschiebung und/oder Temperaturerhöhung freigesetzt werden.

Bei hoher Temperatur im sauren Milieu setzt sich das Addukt aus SO₂ und Inhaltsstoff vollständig um, bis schließlich kein Addukt mehr vorhanden ist. Auf diese Weise kann also das gesamte gebundene SO₂ in freies SO₂ und anteilig in die Gasphase überführt und gemessen werden.

Mit anderen Worten bedeutet dies, daß durch das neue Verfahren die Möglichkeit geschaffen wurde, freie und an Inhaltsstoffe gebundene Komponenten in einer flüssigen Probe in einem einzigen Meßschritt vollständig quantitativ zu erfassen, so daß keine weiteren Messungen von fälschlicherweise miterfaßten Inhaltsstoffen, wie z.B. der Ascorbinsäure erforderlich sind, wie es im Stand der Technik der Fall ist.

Dabei kann der flüssigen Probe vor der Messung ein Reagenz zugegeben werden, das die Bindungsstellen der Inhaltsstoffe für die Komponente absättigt und/oder zerstört.

Auch auf diese Weise kann das Gleichgewicht von dem Addukt zu den Edukten verschoben werden, was insbesondere dann günstig ist, wenn für die Durchführung der eigentlichen Messung Temperaturen verwendet werden müssen, bei denen das Gleichgewicht noch nicht vollständig zu den Edukten verschoben ist.

Allgemein ist es noch bevorzugt, wenn der Aufnahmeraum eine Ablaufleitung und einen Zufluß für eine Reinigungsflüssigkeit aufweist, wobei vorzugsweise der Zufluß mit einer innen im Aufnahmeraum angeordneten Reinigungsdüse verbunden ist.

Nach der erfolgten Messung einer ersten Probe wird diese aus dem Aufnahmeraum abgelassen, der dann mit dem Reinigungsmittel gespült wird. Die Reinigungsdüse verteilt die Reinigungsflüssigkeit dabei in dem gesamten Aufnahmeraum, so daß eine Kontamination der nachfolgend zu vermessenden Probe verhindert wird.

Während der Aufnahmeraum gereinigt und mit einer neuen flüssigen Probe gefüllt wird, die danach noch in beschriebener Weise durch pH-Absenkung und/oder Temperaturerhöhung bzw. Zugabe eines Reagenz vorbereitet wird, wird der Sensor im Standby durch das gefilterte und befeuchtete Trägergas gespült, so daß auch hier sämtliche Verunreinigungen entfernt werden.

Wenn die Probe entsprechend aufbereitet wurde, wird sie im Zirkularbetrieb von dem Trägergas durchspült, so daß sich ein Gleichgewicht zwischen dem gelösten und dem in der Gasphase befindlichen SO₂ einstellt. Danach wird die Ventilschaltung so angesteuert, daß der Sensor mit in den Kreislauf eingeschaltet wird, so daß das Trägergas jetzt den Sensor sowie die flüssige Probe durchspült. In Abhängigkeit von der gemessenen SO₂-Konzentration in der Gasphase kann jetzt anhand von Temperatur, Druck und pH-Wert auf das in der Probe vorhandene, gesamte SO₂ rückgeschlossen werden.

In einer Ausführung wird dabei das durch die Probe geleitete Trägergas in zwei Teile aufgesplittet, von denen der erste im Kurzschluß zurück in die Probe geleitet wird, während der zweite durch den Sensor geht und wahlweise in die Umgebung abgegeben oder ebenfalls zurück in die Probe geleitet wird. Wenn der durch den Sensor geleitete Anteil gering ist, kann auf die Rückführung in die Probe verzichtet werden, ohne daß sich während der Meßzeit merkliche Konzentrationsänderungen ergeben. Vor den Sensor kann dabei ein Massenflußregler geschaltet werden, um einen konstanten Fluß durch den Sensor zu gewährleisten.

Zur Erhöhung der Zuverlässigkeit der Messung kann diese bei verschiedenen Temperaturen und/oder pH-Werten wiederholt und aus der Meßreihe ein entsprechender Mittelwert gebildet werden.

Das Verfahren ist darüber hinaus sehr einfach und standardisiert durchführbar, so daß keine überragenden Fachkenntnisse für die Durchführung des Verfahrens erforderlich sind.

Insgesamt ermöglicht das neue Verfahren, in einer flüssigen Probe Komponenten wie beispielsweise SO₂, aber auch andere, Weinkrankheiten oder Weinfehler hervorrufende Komponenten in Wein oder Fruchtsäften zu bestimmen.

Darüber hinaus kann das neue Verfahren auch verwendet werden, um in anderen, auch festen Lebensmitteln entsprechende Komponenten zu bestimmen. Hierzu ist es lediglich erforderlich, eine Suspension der Festsubstanzen anzufertigen, die dann die flüssige Probe bildet und entsprechend behandelt wird. Auf diese Weise kann der Schwefeldioxidgehalt von z.B. Lebensmitteln bestimmt werden, die mit Schwefeldioxid behandelt wurden.

Von besonderem Vorteil bei dem neuen Verfahren ist zum einen die Verwendung einer elektrochemischen Zelle und weiter die automatisierte Gasführung während der Probenvorbereitung sowie der Messung. Ein besonderer Vorteil ist auch in der Probenaufbereitung, insbesondere in der Überführung von gebundenem in freies SO₂ zu sehen. Diese Probenvorbereitung ist auch ohne die Verwendung des elektrochemischen Sensors für sich genommen neu und erfinderisch, denn sie ist auch mit einem üblichen Gassensor oder einem anderen Meßverfahren durchführbar. Dabei kann für die Absättigung der Bindungsstellen der Inhaltsstoffe für SO₂ ein entsprechendes Reagenz eingesetzt werden.

Bei einer SO₂-Messung wird gemäß dem neuen Verfahren beispielsweise 1 ml der Probe mit einem Reagenz, das eine starke Säure oder eine konzentrierte schwache Säure sein kann und zum Freisetzen des gebundenen SO₂ dient, vermischt, durch einen Durchlauferhitzer geleitet und dann in ein Aufnahmegefäß geführt, in dem ein Verdünnungsmedium bereitgestellt wurde, das beispielsweise 40 ml Wasser oder aber auch eine verdünnte Säure sein kann. Soll freies SO₂ bestimmt werden, wird 1 ml der Probe direkt in das Aufnahmegefäß überführt, in dem eine verdünnte schwache Säure zum Austreiben des SO₂ aus der wäßrigen Phase vorgelegt wird.

Der Inhalt des Aufnahmeraumes wird dann durch einen im Kreislauf geführten Strom von Trägergas begast und auf eine konstante Temperatur gebracht, so daß sich ein temperatur- und druckabhängiges Gleichgewicht einstellt. Nach dem Einstellen des Gleichgewichts wird entweder das gesamte Trägergas oder ein Teil des Trägergases über den Sensor geführt. Das im Bypass geführte Trägergas wird zur Vermeidung von Konzentrationsänderungen in den Aufnahmeraum zurückgeführt, während das durch den Sensor geführte Trägergas auch nach außen entweichen kann.

Selbstverständlich ist es auch möglich, die Probe direkt in ein Reagenz zu dosieren, ggf. auf den Durchlauferhitzer zu verzichten, und die Probe direkt zu begasen, also auf das Verdünnungsmedium zu verzichten, was zu einem kleineren Probenaufnahmeraum führt.

Wenn zwei Reaktoren gleichzeitig verwendet werden, können gebundenes und freies SO₂ zur selben Zeit gemessen werden, was zu einer Zeitersparnis führt.

Durch Kontrolle des Außendruckes und die Berücksichtigung des Druckes bei der Auswertung wird unter bestimmten Bedingungen das Meßergebnis verbessert, weil die Gleichgewichtseinstellung zwischen Flüssigkeit und Gasphase druckabhängig ist. Durch eine möglichst konstante Temperatur kann ebenfalls für eine reproduzierbare Messung gesorgt werden.

Weitere Vorteile ergeben sich aus der Beschreibung und der beigefügten Zeichnung.

Ein Ausführungsbeispiel der Erfindung ist in der Zeichnung dargestellt und wird in der nachfolgenden Beschreibung näher erläutert. Es zeigen:
- Fig. 1: eine vereinfachte schematische Darstellung einer Vorrichtung, die zur Durchführung des erfindungsgemäss Verfahren geeignet ist.
- Fig. 2: eine schematische Darstellung des aktiven Teiles des bei der Vorrichtung aus Fig. 1 verwendeten Sensors; und
- Fig. 3: eine detailliertere schematische Darstellung der Vorrichtung aus Fig. 1.

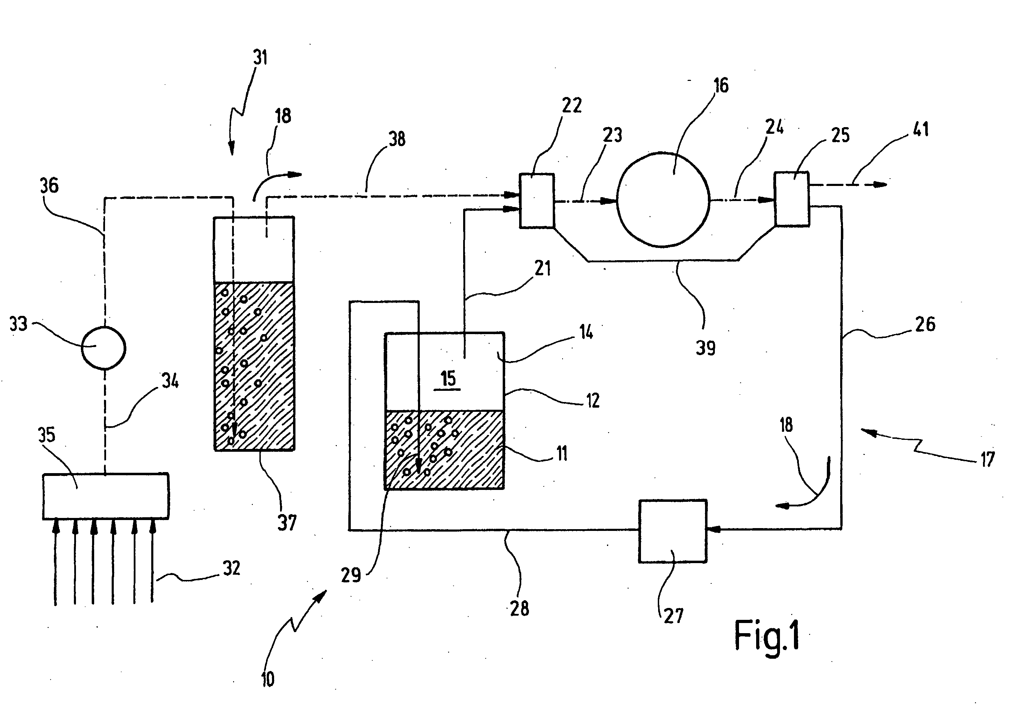

Fig. 1 zeigt eine Vorrichtung 10 zur Bestimmung einer in einer flüssigen Probe 11 frei oder an Inhaltsstoffe gebunden vorhandenen Komponente. Die flüssige Probe 11 ist in einem Probengefäß 12 untergebracht, das einen Aufnahmeraum 14 für die flüssige Probe 11 bildet, über der sich ein Gasraum 15 ausbildet.

Die Messung erfolgt mit einem auf die Komponente ansprechenden Sensor 16. Die flüssige Probe 11 ist Wein, dessen Schwefeldioxidgehalt gemessen werden soll. Schwefeldioxid kommt im Wein sowohl frei als auch an verschiedene Inhaltsstoffe gebunden vor, wobei alle Zustandsformen zusammen die gesamte schwefelige Säure oder SO₂-Konzentration ergeben, die auf bestimmte Maximalwerte hin überwacht wird.

Um das im Wein gelöste Schwefeldioxid zu dem Sensor 16 zu transportieren, ist ein Gasleitungssystem 17 vorgesehen, durch das ein Trägergas 18 im Kreislauf durch die flüssige Probe 11 und den Sensor 16 geleitet wird. Weil der den Sensor verlassende Gasstrom wieder in die Probe rückgeführt wird, besteht während der Messung nicht die Gefahr einer Konzentrationsänderung der zu messenden Komponente in der flüssigen Probe 11.

Das Gasleitungssystem 17 umfaßt eine Leitung 21, die aus dem Gasraum 15 zu einer ersten Ventilschaltung 22 führt. Die Ventilschaltung 22 ist an ihrem Ausgang über eine Leitung 23 mit einem Eingang des Sensors 16 verbunden. Der Ausgang des Sensors 16 führt über eine Leitung 24 zu einer zweiten Ventilschaltung 25, die über eine Leitung 26 an ihrem Ausgang mit einer Pumpe 27 verbunden ist, die das Trägergas 18 im Kurzschluß fördert. Zu diesem Zweck ist die Pumpe 27 an ihrem Ausgang über eine Leitung 28 mit einer Fritte 29 verbunden, durch die das Trägergas 18 großflächig in die flüssige Probe 11 hineingeleitet wird.

Um den insoweit beschriebenen Meß-Kurzschlußkreislauf mit Trägergas 18 zu befüllen, ist das Gasleitungssystem 17 mit einer Gasquelle 31 für das Trägergas 18 verbunden. Während es prinzipiell möglich ist, das Trägergas aus einer Gasflasche bereitzustellen, wird im Ausführungsbeispiel gemäß Fig. 1 hierzu Umgebungsluft 32 angesaugt. Zu diesem Zweck ist eine Pumpe 33 vorgesehen, die an ihrer Ansaugseite über eine Leitung 34 mit einem Filter 35 verbunden ist, das aus der Umgebungsluft 32 die Bestimmung der Komponente störende Anteile herausfiltert. Diese störenden Anteile sind z.B. SO₂, aber auch andere Gase, für die der Sensor 16 eine Querempfindlichkeit aufweist.

Die Pumpe 33 ist an ihrem Ausgang mit einer Leitung 36 verbunden, die in eine Waschflasche 37 führt, in der die gefilterte Umgebungsluft 32 angefeuchtet wird. Aus der Waschflasche 37 gelangt angefeuchtetes Trägergas 18 über eine Leitung 38 in die erste Ventilschaltung 22.

Zwischen den beiden Ventilschaltungen 22, 25 ist noch eine Bypassleitung 39 vorgesehen, über die Trägergas 18 unter Umgehung des Sensors 16 von der Ventilschaltung 22 zu der Ventilschaltung 25 geleitet werden kann. Die zweite Ventilschaltung 25 ist an ihrem Ausgang noch mit einer Abluftleitung 41 verbunden.

Zu Meßbeginn wird durch eine geeignete Stellung der Ventilschaltung 22 und 25 angefeuchtetes Trägergas 18 in den in Fig. 1 mit durchgezogenen Linien dargestellten Teil des Gasleitungssystemes 17 geleitet. Daraufhin werden die Ventilschaltungen 22 und 25 so geschaltet, daß Trägergas im Kreislauf die flüssige Probe 11 begast und mit aufgenommenem SO₂ aus dem Gasraum 15 über die Leitungen 21, 39 und 26 im Kurzschluß transportiert wird. Auf diese Weise wird vor der eigentlichen Messung ein Gleichgewicht zwischen Gasphase und flüssiger Probe 11 eingestellt, so daß der in der folgenden Messung bestimmmte Meßwert unter Anwendung des Henry'schen Gesetzes in die Konzentration der schwefeligen Säure in der flüssigen Probe 11 umgerechnet werden kann.

Parallel zu dieser Gleichgewichtseinstellung wird der Sensor 16 mit dem inerten Trägergas 18 gespült, das aus der Waschflasche 37 kommt und über die Abluftleitung 41 nach außen gegeben wird. Auf diese Weise wird der Sensor 16 vor der eigentlichen Messung mit einem von der zu messenden Komponente sowie bezüglich der Querempfindlichkeit des Sensors 16 weiter störenden Anteilen freien Trägergas gespült, so daß sämtliche Verunreinigungen von dem Sensor 16 entfernt werden, die aufgrund einer vorhergehenden Messung dort ggf. noch vorhanden sind.

Wenn der Sensor 16 hinreichend lange gespült wurde und sich im übrigen in dem über die Bypassleitung 39 eingestellten Kurzschluß-Kreislauf ein entsprechendes Gleichgewicht zwischen Gasphase und Probe 11 eingestellt hat, werden die Ventilschaltungen 22, 25 so umgeschaltet, daß der Sensor 16 jetzt in diesen Kreislauf eingeschaltet wird. Weil auch während der Messung der Kreislauf erhalten bleibt, bleibt die in der Gasphase, also im Gasraum 15 vorhandene Konzentration der zu messenden Komponente, im Falle von Wein als flüssiger Probe 11 also z.B. SO₂, konstant. Der von dem Sensor 16 abgegebene Meßwert läßt sich folglich mit dem Henry'schen Gesetz in die Konzentration von SO₂ im Wein umrechnen.

Die Ventilschaltung 22 kann dabei auch so ausgelegt sein, daß sie nach der Einstellung des Gleichgewichtes, wo das Trägergas 18 ausschließlich durch die Bypassleitung 39 fließt, nur einen Teil des Trägergases 18 für den Fluß durch den Sensor 16 abzweigt, so daß weiterhin Trägergas 18 durch die Bypassleitung 39 zurück in den Aufnahmeraum 14 geleitet wird. Das durch den Sensor 16 geleitete Trägergas kann dabei durch die Ventilschaltung 25 entweder auch zurück in den Aufnahmeraum 14 geleitet werden, oder aber über die Abluftleitung 41 nach außen gegeben werden.

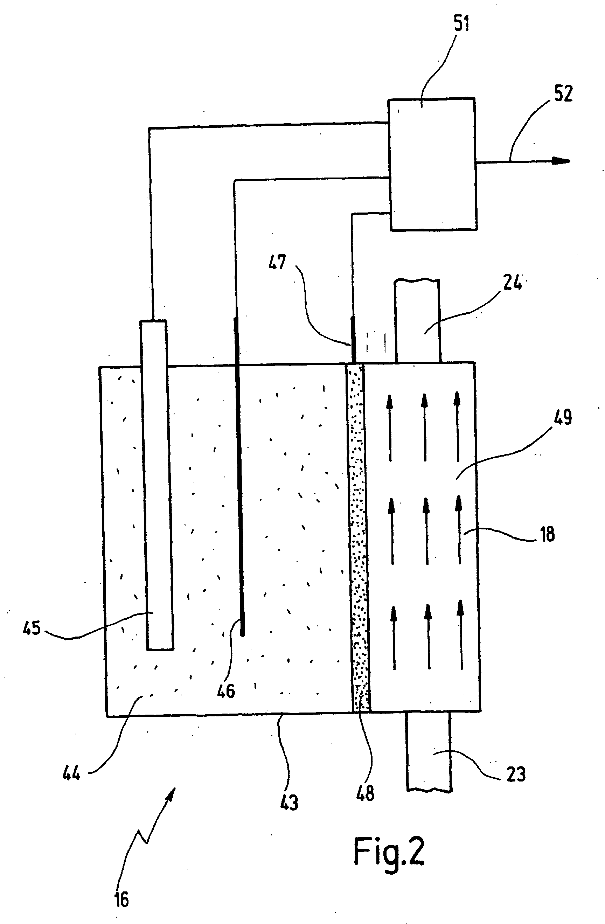

Fig. 2 zeigt in einer schematischen Darstellung den Sensor 16, der hier eine elektrochemische Zelle ist, wie sie beispielsweise von Hodgson et al., a.a.O. oder Schiavon und Zotti, a.a.O., beschrieben wurde.

Gemäß der schematischen Darstellung der Fig. 2 umfaßt der Sensor 16 einen Elektrolytraum 43, in dem sich ein geeigneter Elektrolyt 44 befindet. In den Elektrolyten 44 ist eine Referenzelektrode 45 sowie eine Gegenelektrode 46 eingetaucht. Ferner ist eine Arbeitselektrode 47 vorgesehen, die auf einer Membran 48 mit Schwefeldioxid-sensitiver Beschichtung angebracht ist.

Die Membran 48 trennt den Elektrolytraum 43 von einem Gasstromraum 49, den das Trägergas 18 parallel zur Membran 48 durchströmt. Der Gasstromraum 49 ist mit den Leitungen 23 und 24 verbunden.

Die Elektroden 45, 46 und 47 sind mit einer Potentiostatenschaltung 51 verbunden, die an ihrem Ausgang 52 ein Meßsignal liefert, das kennzeichnend für den Schwefeldioxidgehalt in dem Trägergas 18 ist und in die Schwefeldioxidkonzentration in der flüssigen Probe 11 umgerechnet werden kann.

Eine prinzipielle Schaltung zur Ansteuerung des Sensors 16 wird von Kissinger und Heinemann beschrieben in "Laboratory Techniques in Electroanalytical Chemistry", 2. Ausgabe, 1996, Marcel Dekker Inc., New York, Basel, Hongkong.

Wie aus der Literatur allgemein bekannt, hängt die Gasspezifität des Sensors 16 vom Elektrolyten, dem eingestellten Redoxpotential sowie der aktiven Sensoroberfläche ab. Über die Potentiostatenschaltung 51 wird das Potential an der Arbeitselektrode 47 gegenüber der Referenzelektrode 46 eingestellt, um den Stromfluß durch die Zelle messen zu können. Zur Einstellung des Potentials des Elektrolyten 44 ist die Gegenelektrode 46 vorgesehen, die den Gegenstrom der Arbeitselektrode 47 trägt.

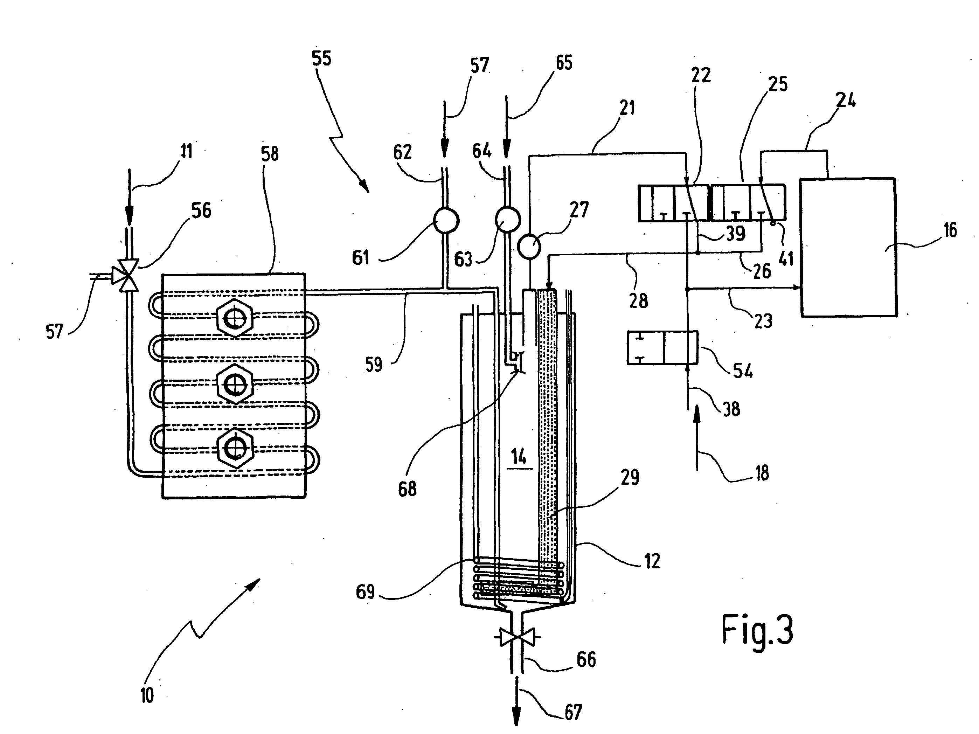

In Fig. 3 ist die Vorrichtung 10 aus Fig. 1 im Ausschnitt, aber detaillierter dargestellt, wobei die Pumpe 27 nicht eingangs der Fritte 29 sondern ausgangs des Gasraumes 15 angeordnet ist.

Wie bereits anhand von Fig. 1 beschrieben, ermöglichen die Ventilschaltungen 22, 25 parallel den Spülbetrieb des Sensors 16 und den Kreislaufbetrieb mittels der Bypassleitung 39. Dieser Zustand ist in Fig. 3 gezeigt.

Durch Umschalten der Ventilschaltungen 22, 25 sowie durch Schließen des Ventiles 54, über das Trägergas 18 aus der Waschflasche 37 kommt, wird der Sensor 16 in den Kreislauf eingeschaltet, so daß die in dem Trägergas 18 vorhandene Komponente gemessen werden kann.

Zur Befüllung des Probengefäßes 12 mit flüssiger Probe 11 ist ein Probenleitungssystem 55 vorgesehen, über das flüssige Probe 11 in den Aufnahmeraum 14 einfüllbar ist. Am Einlaß des Probenleitungssystemes 55 befindet sich ein Dreiwegehahn 56, über den flüssige Probe 11 mit einem geeigneten Verdünnungsmedium 57, vorzugsweise Wasser, verdünnt wird. An den Dreiwegehahn 56 schließt sich eine Heiz-/Kühleinrichtung 58 an, in der die flüssige Probe auf die für die Messung erforderliche Temperatur erwärmt wird. Über eine Probenleitung 59 gelangt die mit dem Verdünnungsmedium 57 verdünnte flüssige Probe 11 dann in den Aufnahmeraum 14.

An die Probenleitung 59 ist über eine Pumpe 61 ein Einlaß 52 für weiteres Verdünnungsmedium 57 angeschlossen, über das z.B. der pH-Wert der flüssigen Probe 11 im Aufnahmeraum 14 verändert werden kann.

Über eine weitere Pumpe 63 kann mittels eines Einlasses 64 Reinigungsmittel 65 in den Aufnahmeraum 14 geleitet werden, wobei eine Entleerung des Aufnahmeraumes 14 über einen Ablauf 66 erfolgt, aus dem nach einer Messung die flüssige Probe 11 als Abfall 67 abgelassen wird.

In dem Probengefäß 12 ist noch eine Heizwendel 69 angedeutet, über die die flüssige Probe 11 in dem Aufnahmeraum 14 vor der Messung erhitzt werden kann.

Durch eine Erhitzung der flüssigen Probe 11 vor der eigentlichen Messung und durch eine Absenkung des pH-Wertes in den sauren Bereich wird zur Freisetzung des Schwefeldioxids beigetragen. An Inhaltsstoffe der flüssigen Probe 11 gebundenes Schwefeldioxid kann auf diese Weise freigesetzt werden. Bei hoher Temperatur im sauren Bereich setzt sich nämlich das Addukt aus SO₂ und Inhaltsstoff vollständig um, bis schließlich kein Addukt mehr vorhanden ist. Auf diese Weise kann also das gesamte gebundene SO₂ in freies SO₂ und anteilig in die Gasphase überführt und dort gemessen werden.

Es ist auch möglich, über das Verdünnungsmedium 57 den pH-Wert der flüssigen Probe 11 einzustellen und diese dann in der Heiz/Kühleinrichtung 58 entsprechend zu erhitzen, um das Addukt weiter in die Edukte zu überführen. Die so aufbereitete Probe 11 wird dann in eine definierte Menge Wasser in dem Aufnahmeraum 14 geleitet und wie beschrieben vermessen. Auf diese Weise wird nur ein Teil der im Aufnahmeraum 14 aufzunehmenden Flüssigkeitsmenge erhitzt, die Temperatur im Probengefäß 12 selbst kann geringer gehalten werden. Ferner wird so unter Umständen vermieden, daß die zur Probenaufbereitung im Probengefäß 12 stark erhitzte Flüßigkeit vor der Messung wieder abgekühlt werden muß.

Wenn der flüssigen Probe 11 vor der Messung beispielsweise über den Einlaß 62 ein entsprechendes Reagenz zugegeben wird, kann das Gleichgewicht von dem Addukt zu den Edukten hin verschoben werden, was insbesondere dann angewendet wird, wenn für die Durchführung der Messung Temperaturen verwendet werden müssen, bei denen das Gleichgewicht noch nicht vollständig zu den Edukten verschoben ist.

Die Versorgung mit Reinigungsmittel 65 kann z.B. über einen "Hausanschluß" mit deionisiertem Wasser erfolgen, das direkt über einen Druckregler zu der Reinigungsdüse 68 geführt werden kann. Wenn kein deionisiertes Wasser im Hausanschluß vorhanden ist, gibt es die Möglichkeit, über einen Druckminderer und eine zwischengeschaltete Ionenaustauscher-Kartusche Reinigungsmittel 65 direkt aus der Hauswasserleitung zu erzeugen und einzuspeisen. Auf diese Weise kann der erforderliche hohe Druck von ca. 2 bar und das zur Reinigung notwendige Fördervolumen von 1 bis 3 l/min auf preiswerte Weise erreicht werden, ohne daß destilliertes Wasser manuell in Kanistern beschafft werden muß.

## Patentansprüche

1. Verfahren zur Bestimmung des (11) frei oder an Inhaltsstoffe gebundenen SO₂ in Wein (11), bei dem aus einem Gasraum (15) über dem Wein (11) das SO₂ in der Gasphase gemessen wird,
**dadurch gekennzeichnet, daß** der Wein (11) mit einem Trägergas (18) begast wird, das im Kurzschluß durch den Wein (11) geleitet wird, um einen Gleichgewichtszustand einzustellen, und daß die Konzentration des in der Gasphase befindlichen SO₂ mittels eines Sensors, vorzugsweise einer elektrochemischen Zelle (16) erfaßt und in ein elektrisches Signal umgewandelt wird.

2. Verfahren nach Anspruch 1, **dadurch gekennzeichnet, daß** das Trägergas (18) im Kurzschluß durch den Wein (11) und die elektrochemische Zelle (16) geleitet wird.

3. Verfahren nach Anspruch 2, **dadurch gekennzeichnet, daß** das Trägergas (18) zu einem vorzugsweise kleinen Teil durch den Sensor (16) geleitet wird und das restliche Trägergas (18) im Kurzschluß zurück durch den Wein (11) geleitet wird.

4. Verfahren nach einem der Ansprüche 1 bis 3, **dadurch gekennzeichnet, daß** die elektrochemische Zelle (16) vor der eigentlichen Messung mit einem Trägergas (18) gespült wird.

5. Verfahren nach Anspruch 4, **dadurch gekennzeichnet, daß** das Trägergas (18) befeuchtet wird.

6. Verfahren nach einem der Ansprüche 3 bis 5, **dadurch gekennzeichnet, daß** aus dem Trägergas (18) eventuell vorhandene Anteile des SO₂ ausgefiltert werden.

7. Verfahren nach einem der Ansprüche 1 bis 6, **dadurch gekennzeichnet, daß** der Wein (11) vor der Messung erhitzt wird.

8. Verfahren nach einem der Ansprüche 1 bis 7, **dadurch gekennzeichnet, daß** der pH-Wert des Weins (11) vor der Messung in den sauren Bereich abgesenkt wird.

9. Verfahren nach einem der Ansprüche 1 bis 8, **dadurch gekennzeichnet, daß** dem Wein (11) vor der Messung ein Reagenz zugegeben wird, das die Bindungsstellen der Inhaltsstoffe für das SO₂ absättigt und/oder zerstört.

## Claims

1. A method for determining SO₂ that is present in wine (11) in free state or bound to constituents, in which method SO₂ is being measured in the gas phase from a gas space (15) over the wine (11),
**characterized in that** the wine (11) is gazed with a carrier gas (18) that is passed in short circuit through the wine (11) to establish an equilibrium state, and that the concentration of SO₂ present in the gas phase is measured by means of a sensor, preferably an electrochemical cell (16), and is converted into an electrical signal.

2. The method of claim 1, **characterized in that** the carrier gas (18) is passed in short circuit through the wine (11) and the electrochemical cell (16).

3. The method of claim 2, **characterized in that** a preferably small portion of the carrier gas (18) is passed through the sensor (16) and that the remaining carrier gas (18) is passed in short circuit back through the wine (11).

4. The method of anyone of claims 1-3, **characterized in that** the electrochemical cell (16) is purged with a carrier gas (18) before the actual measurement.

5. The method of claim 4, **characterized in that** the carrier gas (18) is moistened.

6. The method of anyone of claims 3-5, **characterized in that** any contents of SO₂ possibly present are filtered out of the carrier gas (18).

7. The method of anyone of claims 1-6, **characterized in that** the wine (11) is heated prior to the measurement.

8. The method of anyone of claims 1-7, **characterized in that** the pH-value of the wine (11) is lowered to the acidic range prior to the measurement.

9. The method of anyone of claims 1-8, **characterized in that** prior to the measurement there is added to the wine (11) a reagent which saturates and/or destroys the binding sites of the constituents for SO₂.

## Revendications

1. Procédé pour déterminer le SO₂ libre ou lié à des composants contenu dans le vin (11), dans lequel le SO₂ est mesuré en phase gazeuse à partir d'une enceinte gazeuse (15) au-dessus du vin (11),
**caractérisé en ce que** le vin (11) est vaporisé avec un gaz porteur (18) qui est passé en circuit fermé dans le vin (11) pour établir un état d'équilibre, et **en ce que** la concentration en SO₂ se trouvant en phase gazeuse est captée au moyen d'un capteur, de préférence une cellule électrochimique (16) et convertie en un signal électrique.

2. Procédé selon la revendication 1, **caractérisé en ce que** le gaz porteur (18) est passé en circuit fermé dans le vin (11) et la cellule électrochimique (16).

3. Procédé selon la revendication 2, **caractérisé en ce que** le gaz porteur (18) est conduit dans une proportion de préférence faible dans le capteur (16) et **en ce que** le reste du gaz porteur (18) est repassé dans en circuit fermé dans le vin (11).

4. Procédé selon l'une quelconque des revendications 1 à 3, **caractérisé en ce que** la cellule électrochimique (16) est lavée avant la mesure véritable avec un gaz porteur (18).

5. Procédé selon la revendication 4, **caractérisé en ce que** le gaz porteur (18) est humidifié.

6. Procédé selon l'une quelconque des revendications 3 à 5, **caractérisé en ce que** d'éventuelles parts existantes de SO₂ sont filtrées hors du gaz porteur (18).

7. Procédé selon l'une quelconque des revendications 1 à 6, **caractérisé en ce que** le vin (11) est chauffé avant la mesure.

8. Procédé selon l'une quelconque des revendications 1 à 7, **caractérisé en ce que** le pH du vin (11) avant la mesure est abaissé dans le domaine acide.

9. Procédé selon l'une quelconque des revendications 1 à 8, **caractérisé en ce qu'**on ajoute au vin (11) avant la mesure un réactif qui sature et/ou détruit les liaisons des composants pour le SO₂.
